# EUROPÄISCHE PATENTSCHRIFT

(11) **EP 2 756 825 B1**
(45) Veröffentlichungstag und Bekanntmachung des Hinweises auf die Patenterteilung: **24.04.2019**
(21) Anmeldenummer: 13195647.6
(22) Anmeldetag: 04.12.2013
(51) Int. Cl.: A61F 2/46, A61F 2/30

(54) **Ausschlagwerkzeug für minimalinvasive Prothesenrevision**
Drift for minimally invasive revision of prostheses
Outil à choc pour une révision de prothèse mini-invasive

(30) Priorität: 22.01.2013 DE 102013200924
(43) Veröffentlichungstag der Anmeldung: 23.07.2014
(73) Patentinhaber: Müller, Erich Johann, 63839 Kleinwallstadt (DE)
(72) Erfinder: Müller, Erich Johann, 63839 Kleinwallstadt (DE)
(74) Vertreter: Becker Kurig Straus

(56) Entgegenhaltungen:
- EP-A2- 0 333 990
- WO-A1-93/25164
- WO-A1-2007/062103
- WO-A1-2007/068219
- WO-A1-2008/028451
- DE-A1- 10 013 331
- US-A- 5 582 084
- US-A1- 2010 123 325
- US-B1- 6 322 564
- US-B1- 8 262 667

## Beschreibung

Die Erfindung betrifft chirurgische Werkzeuge zum Einsetzen und Ausschlagen von Gelenkprothesen.

### Stand der Technik

Seit vielen Jahrzehnten werden künstliche Gelenke und Gelenkprothesen chirurgisch eingesetzt, beispielsweise Hüftgelenksprothesen, Schulterprothesen oder Knieprothesen. So ist die Prothese des Hüftgelenks die am häufigsten durchgeführte Gelenkersatzoperation; allein in Deutschland werden etwa 200.000 Hüftgelenkendoprothesen jährlich implantiert; mehr als 175.000 Menschen werden jährlich infolge eines Gelenkverschleißes am Kniegelenk operiert. Allerdings sind die Prothesen nur beschränkt haltbar und müssen daher, insbesondere wegen aseptisch gelockerter Implantate, nach einer gewissen Zeit ausgewechselt werden. Im Fall einer Hüftprothese beträgt die Haltbarkeit heute beispielsweise 10-15 Jahre. Die Revisionseingriffsrate in den ersten zehn Jahren nach Implantation liegt bei 10%.

Dennoch sind selbst gelockerte Prothesen oder Prothesenteile üblicherweise noch sehr fest mit den jeweiligen Knochenstrukturen verbunden und verzahnt. Es ist daher immer noch problematisch, eine Prothese aus dem Körper zu entfernen, ohne dabei Folgeschäden am umliegenden Knochenlager oder an sonstigem Gewebe des Patienten zu verursachen. Ähnliche Bedingungen sind auch beim Einsetzen neuer Prothesen vorhanden. Außerdem ist es für den Patienten vorteilhaft, den Eingriff möglichst gewebeschonend durchzuführen, so dass minimalinvasive Operationsverfahren (Minimal Invasive Surgery, MIS) mit möglichst kleiner Eröffnung bzw. Verletzung von Haut und Gewebeteilen wünschenswert sind und mittlerweile sehr gebräuchlich sind. Positive Effekte sind die Schonung der Muskelansätze, der Erhalt der Muskelfunktionen, postoperativ reduzierte Schmerzsymptome und eine schnellere Rehabilitation des Patienten. Allerdings werden dadurch neue hohe Anforderungen an die verwendeten Instrumente beim minimalinvasiven Einsetzen und Ausschlagen von Prothesen gestellt.

Die Kräfte beim Ausschlagen einer Prothese müssen also hoch sein, gleichzeitig aber sehr gezielt übertragen werden, so dass sie nur auf den Prothesenschaft/-hals wirken. Ein sehr hoher Impuls ist notwendig, um die Verbindung zwischen Prothese und Knochengewebe zu lösen, bevor die Energie auf den Knochen übertragen werden kann. Dazu ist eine möglichst axiale Kraftübertragung mit exakter Positionierung der Werkzeuge notwendig.

Bisherige Ausschlagwerkzeuge sind oft nur für eine Prothesenart anwendbar, teilweise sogar nur für Prothesen einer speziellen Bauweise bzw. eines Herstellers. Außerdem ist die Positionierung und Fixierung des Werkzeugs, wie vorstehend beschrieben, nicht einfach zu erreichen.

Aus US 6322564 B1, WO 2008/028451 A1, US8262667B1, WO 2007/062103 A1 und EP 0333990 A2 sind verschiedene Klemm- bzw. Halteinstrumente für Prothesen bekannt, die alle nach einem Scheren- bzw. Zangenprinzip mit überkreuzten Klemmhebeln funktionieren. Außerdem sind aus DE 10013331 A1 und WO 93/25164 Halteinstrumente bekannt, bei denen die Klemmbacken über Druck oder Zug einer über ein Zwischenstück verbundenen Antriebsstange bewegt werden. US 2010/0123325 A1 offenbart eine Greifvorrichtung, bei welcher Zangenelemente, die mit einem Werkstück in Eingriff gebracht werden können, pneumatisch gesteuert zwischen einer Aktivierungs- und einer Deaktivierungsposition bewegt werden können. US 5,582,084 betrifft eine verlängerte Greifzange, welche es ermöglicht, schwer zugängliche Bereiche, z.B. eines Motors, zu erreichen. WO93/25164 zeigt ein Werkzeug zum Einsetzen der Femurkomponente mit zwei Spannbacken, die durch Axialbewegung eines Stellelements entlang der Werkzeuglängsachse aufeinander zu bewegbar sind.

### Kurze Zusammenfassung der Erfindung

Es ist daher Aufgabe der Erfindung, ein chirurgisches Werkzeug zum Ausschlagen und/oder Einsetzen von Gelenkprothesen bereitzustellen, das eine schonende Entfernung der Prothesenteile für unterschiedliche Gelenke bei minimalinvasiven Eingriffen erlaubt.
Diese Aufgabe wird gelöst durch einen Werkzeugkopf und ein Werkzeug nach den beigefügten Ansprüchen.

Ein Werkzeug gemäß der Erfindung umfasst im wesentlichen zwei Teile; einen Werkzeugkopf, der zum Ausschlagen der Prothese dient, und einen Bedienteil, der zur Betätigung des Werkzeugkopfes dient. Auf diese Weise erfolgen alle Schritte zur Betätigung des Werkzeugs außerhalb des Operationsfelds, was zum einen eine verbesserte Bedienbarkeit für den Operateur ermöglicht, vor allem aber auch minimalinvasive Eingriffe erlaubt, da keine ausladenden Bedienelemente wie Schrauben oder Hebel am Werkzeugkopf vorhanden sein müssen. Durch die Spannbacken am Werkzeugkopf, die über entfernte Bedienelemente am Bedienteil und nicht direkt am Werkzeugkopf betätigt werden, kann eine Prothese oder ein Prothesenteil auf einfache Weise eingespannt und sicher fixiert werden. Nach dem Einspannen des Prothesenteils kann dann ein gezielter axialer Impuls zum Ausschlagen oder Einsetzen der Prothese über das Bedienteil aufgebracht werden. Die komfortable Positionierung ermöglicht ein schonendes Ausschlagen mit minimalen Schädigungen des umliegenden Gewebes. Außerdem kann das Werkzeug modular aufgebaut sein, so dass jedes Bedienteil mit unterschiedlichsten Werkzeugköpfen kombiniert werden kann. Auf diese Weise erhält man ein vielseitiges Ausschlagwerkzeug, das optimal an die speziellen Prothesenbedingungen und die notwendigen Kräfte angepasst werden kann.

Die Erfindung umfasst einen Werkzeugkopf für ein chirurgisches Werkzeug zum Ausschlagen und/oder Einsetzen von Protheses gemäss des unabhängigen Anspruchs 1.

Bei einem solchen Werkzeugkopf kann mittels eines entsprechenden Bedienteils die Druckausübung auch indirekt und damit außerhalb des Operationsfelds in gewünschtem Abstand stattfinden, was minimalinvasive Operationen ermöglicht.

Die Spannbacken können gerade oder überkreuzt angeordnet sein. Bei Überkreuzung können die mindestens zwei Spannbacken 14c, 15a, 15b an ihrem Kreuzungspunkt durch ein Stiftgelenk 13b verbunden sein.

Nach einer weiteren Ausführungsform kann mindestens eine der Spannbacken 14, 14a, 14b, 15a relativ zur Werkzeugachse seitlich abgewinkelt sein, um beispielsweise einen vorteilhafteren Kraftvektor zu erreichen.

In speziellen Ausführungsformen kann mindestens eine der zangenartigen Spannbacken mehrere Abschnitte umfassen, die jeweils durch ein Stiftgelenk 13a schwenkbar miteinander verbunden sind.

In einem illustrativen Beispiel, das nicht unter den Schutzbereich des Anspruchs 1 fällt, weist der Werkzeugkörper 10, 11 eine Gleitschiene oder Führung 50 auf, in der eine bewegliche Spannbacke 41 verschiebbar gelagert ist, wobei eine zweite Spannbacke durch einen Endbereich des Werkzeugkörpers 10, 11 gebildet wird. Der Endbereich des Werkzeugkörpers kann dann eine Öffnung 55, 55a zur Aufnahme eines Prothesenteils aufweisen, wobei die bewegliche Spannbacke 41 so angeordnet ist, dass sie in die Öffnung hinein bewegt werden kann. Die Öffnung kann eine seitliche Öffnung oder ein Durchgangsloch sein, wobei die Öffnung so bemessen ist, dass das gewünschte Prothesenteil - etwa ein Prothesenhals darin aufgenommen werden kann. In der Gleitschiene des Werkzeugkörpers abgewandt vom Endbereich und angrenzend an die bewegliche Spannbacke kann außerdem ein Stellglied verschiebbar eingesetzt sein, das so angeordnet ist, dass es Druck auf die bewegliche Spannbacke ausüben kann.

In allen Ausführungsformen des erfindungsgemäßen Werkzeugkopfes kann optional mindestens ein Rückstellelement 18, beispielsweise in Form einer Feder, angeordnet sein, welches die Spannbacken 14, 14a, 14b, 14c, 15, 15a, 15b, 41 bei Beendigung der Druckausübung in ihre ursprüngliche Position zurückversetzt.

Um den Halt zu verbessern, kann optional in beliebigen Ausführungsformen mindestens eine der Spannbacken 14, 14a, 14b, 14c, 15, 15a, 15b, 41 in einem Haltebereich, der im Einsatz an einem einzuspannenden Prothesenteil anliegt, mit einem Profil 17 versehen sein, etwa mit Einkerbungen oder Rillen.

Die Erfindung betrifft außerdem ein chirurgisches Werkzeug zum Ausschlagen und/oder Einsetzen von Prothesen, umfassend einen Werkzeugkopf wie vorstehend beschrieben und einen Bedienteil, wobei das Bedienteil ein Führungsgehäuse 20, 29, 30 mit einer axialen Ausnehmung umfasst, die mindestens in Richtung des Werkzeugkopfes geöffnet ist; eine in dem Führungsgehäuse 20, 29, 30 axial verschiebbar gelagerte Stellstange 43, 43a; ein Bedienelement 28; und eine Schlagvorrichtung 30a, 29a, 25, 26, die dazu eingerichtet ist, einen auf das Bedienteil angewandten Impuls aufzunehmen oder aufzubringen und über das Werkzeug auf die Prothese zu übertragen; wobei der Werkzeugkörper 10, 11, 12 des Werkzeugkopfes an dem Führungsgehäuse 20, 29, 30 des Bedienteils befestigt ist; dadurch gekennzeichnet, dass das Bedienelement 28 so angeordnet ist, dass eine Betätigung des Bedienelements 28 eine Bewegung der Stellstange 43, 43a in Richtung des Werkzeugkopfes bewirkt, wobei die Stellstange so angeordnet ist, dass sie bei Betätigung einen Druck auf den Druckbereich 16 der mindestens einen beweglichen Spannbacke 14, 14a, 14b, 14c, 15, 15a, 15b, 41 ausübt.

Die Schlagvorrichtung kann beispielsweise eine Schlagplatte 29a zum Aufnehmen eines mechanischen Schlags am werkzeugkopfabgewandten Ende des Bedienteils umfassen; oder einen Gleithammer 25, der beweglich auf dem Führungsgehäuse 20 gelagert ist, und einen Anschlag 26 für den Gleithammer an einem oder beiden Enden des Führungsgehäuses; oder einen Pneumatikadapter, um ein pneumatisches Werkzeug anzuschließen. Das Bedienelement 28 kann beispielsweise einen Exzenterhebel umfassen.

Vorzugsweise ist der Werkzeugkopf lösbar durch ein Gewinde an dem Bedienteil befestigt.

### Zeichnungen

Die Erfindung wird nachstehend unter Bezug auf die Figuren ausführlicher beschrieben, wobei
Figur 1 eine perspektivische Darstellung eines erfindungsgemäßen Werkzeugs mit Werkzeugkopf und Bedienteil nach dem Gleithammerprinzip zeigt;
Figur 2 einen Querschnitt durch ein Werkzeug wie in Figur 1 darstellt;
Figur 3a einen Querschnitt durch eine weitere Ausführungsform eines erfindungsgemäßen Werkzeugs mit einem Bedienteil mit Pneumatikadapter zeigt, und Figur 3b dasselbe Bedienteil alleine ohne Werkzeugkopf darstellt;
Figur 4a ein illustratives Beispiel eines Werkzeugs mit Ausschlagplatte und einem Werkzeugkopf mit einer Hüftgelenksprothese darstellt, wobei Figur 4b ein entsprechendes Werkzeug im Querschnitt zeigt;
Figuren 5, 6, 7, 8, 9 und 10 jeweils verschiedene beispielhafte Werkzeugköpfe mit Zangenfunktion für verschiedene Einsatzbereiche zeigen;
Figur 11 ein weiteres Werkzeug mit Gleithammer-Bedienteil und zangenartigem Werkzeugkopf zeigt; und
Figur 12a eine weitere Ausführungsform eines Werkzeugs mit Bedienteil zeigt, wobei Figur 12b nur einen entsprechenden Werkzeugkopf alleine detaillierter zeigt.

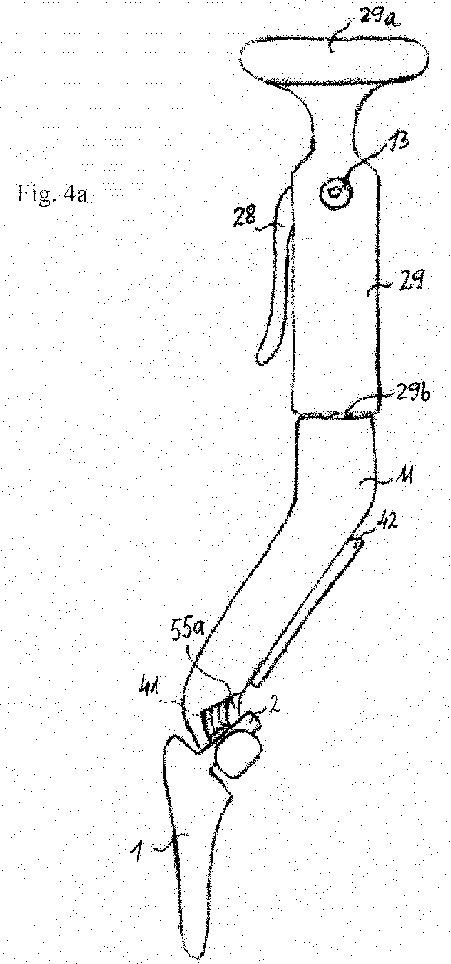

Die Figuren 4a, 4b, 8, 9 und 10 zeigen illustrative Beispiele, die nicht unter den Schutzbereich der Ansprüche fallen.

### Beschreibung beispielhafter Ausführungsformen

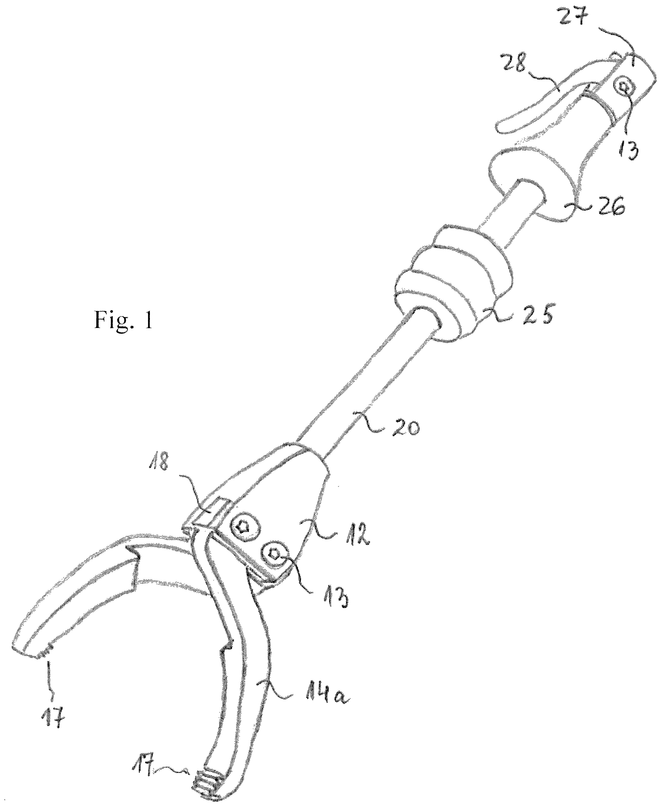

In Figur 1 ist eine mögliche Ausführungsform eines erfindungsgemäßen Werkzeugs gezeigt. Der Werkzeugkopf umfasst einen Werkzeugkörper 12 und zwei zangenartige Spannbacken 14a, die in diesem Ausführungsbeispiel über zwei Stifte oder Bolzen 13 beide beweglich an dem Werkzeugkörper 12 befestigt sind. Die Spannbacken werden bei Benutzung an die Prothese angelegt und mittels des Bedienteils, wie nachstehend beschrieben wird, aufeinander zu bewegt, so dass sie die Prothese mindestens im Haltebereich 17 greifen und einspannen können. Im gezeigten Beispiel weist der Haltebereich 17 außerdem Einkerbungen oder Rillen auf, die einen verbesserten Eingriff mit dem einzuspannenden Prothesenteil sicherstellen. Anstelle der Einkerbungen könnten auch andere Profilierungen, wie etwa Spitzen oder Riffelungen, in mindestens einem der Haltebereiche aufgebracht sein, oder, falls der Anpressdruck auch bei glattem Werkzeug ausreichend ist, vollständig weggelassen werden. Die Spannbacken 14a sind überkreuzt angeordnet und gekrümmt geformt. Dabei weisen die Spannbacken natürlich solche Abmessungen auf, dass sie an den Durchmesser und die nutzbaren Halteflächen des einzuspannenden Prothesenteils, etwa eines Prothesenhalses, angepasst sind. Eine Blattfeder 18 kann im Bereich des Werkzeugkörpers 12 an einer oder beiden Spannbacken 14a so angebracht sein, dass sie bei Verminderung der Stellkraft auf die Spannbacken selbige in die ursprüngliche Position zurückführen, um das Werkzeug wieder von der Prothese zu entfernen.

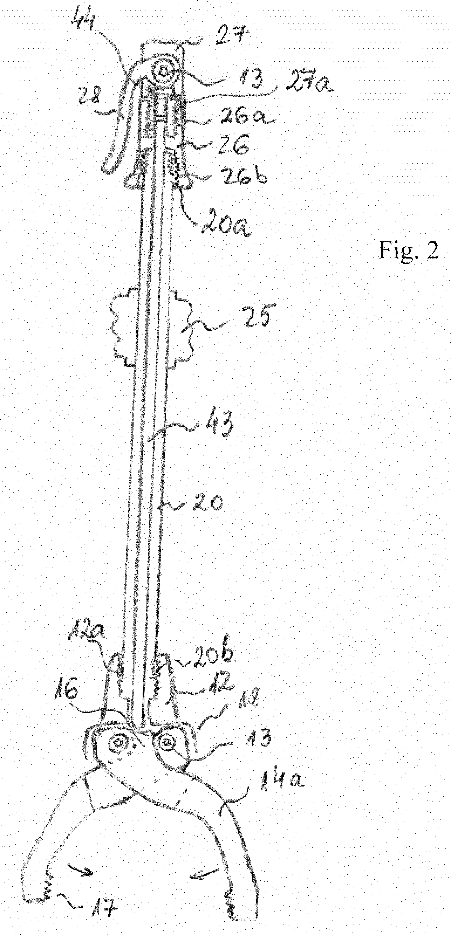

An dem Werkzeugkörper 12 ist der Bedienteil des Werkzeugs angebracht. Dieser umfasst in diesem Beispiel eine Führungsstange 20, ein gleitend auf dieser Stange gelagertes Hammerteil 25, einen Haltegriff 26, und ein Stellelement 28 in Form eines Exzenterhebels. Der Exzenterhebel ist mittels eines Scharniers oder Stifts 13 mit dem Haltegriff verbunden. Nicht sichtbar ist eine Stellstange, die innerhalb der Führungsstange 20 verläuft und in Verbindung mit Figur 2 näher beschrieben wird. Die Stellvorrichtung aus Stellstange und Stellelement bzw. Hebel ermöglicht eine Betätigung der zangenartigen Spannbacken, so dass bei Bedienung des Hebels 28 die beiden Spannbacken 14a aufeinander zu bewegt werden und das Werkzeug einspannen. Der Gleithammer ermöglicht dann einen Impulsübertrag auf das eingespannte Prothesenteil, indem das Hammerteil gleitend auf der Führungsstange 20 nach oben oder unten bis zu einem Anschlag bewegt wird. Auf diese Weise kann das Prothesenteil zwischen den Spannbacken fixiert und mittels Gleithammer ausgeschlagen werden.

Figur 2 zeigt das beispielhafte Werkzeug aus Figur 1 im Querschnitt. Hier wird die Funktionsweise des Stellglieds deutlich. Wieder sieht man den Werkzeugkopf mit dem Werkzeugkörper 12, an dem mit Stiften 13 die beiden Spannbacken 14a mit den eingekerbten Haltebereichen 17 beweglich befestigt sind. Der Werkzeugkopf ist über Gewinde 12a bzw. 20b mit der Führungsstange 20 lösbar verbunden, auf der das Hammerstück 25 gleiten kann. Die Führungsstange weist innen eine Durchführung auf, in der die Stellstange 43 verläuft. Am vom Werkzeugkopf abgewandten Ende der Führungsstange ist der Haltegriff 26 des Gleithammers mittels der Gewinde 26b bzw. 20a befestigt. Darüber ist, ebenfalls über Gewinde, das Spannteil 27 angebracht, an dem der Exzenterhebel 28 als Stellelement über einen Stift 13 beweglich befestigt ist. Zwischen dem Hebel 28 und der Stellstange 43 sorgt ein Druckstück 44 für den Kraftübertrag. Ebenso könnte aber in einer anderen Ausführungsform der Exzenterhebel 28 direkt auf die Stellstange 43 einwirken.

Die Stellstange 43 und die Spannbacken 14a sind so angeordnet, dass eine Bewegung der Stellstange 43 nach unten in Richtung Werkzeugkopf einen Druck auf einen Pressbereich oder Pressflügel 16 der Spannbacken ausübt, wobei der Pressbereich direkt unter der Stellstange angeordnet ist. Der über die Stellstange 43 nach unten ausgeübte Druck bewirkt in dieser Ausführungsform aufgrund der gebogenen und überkreuzten Form der Spannbacken 14a, dass die Spannbacken und damit ihre Haltebereiche aufeinander zu bewegt werden. Wenn also der Hebel 28 gespannt wird, wird dadurch die Stellstange in Richtung Werkzeugkopf gedrückt und die Spannbacken aufeinander gepresst, so dass ein dazwischen befindliches Prothesenteil eingespannt wird. Nachdem ein Prothesenteil auf diese Weise über die Spannvorrichtung fixiert wurde, wird der Gleithammer auf übliche Weise angewendet, um die Prothese auszuschlagen, indem das Hammerteil nach oben gegen den Anschlag des Griffteils 26 ausgelenkt wird. Ebenfalls in Figur 2 sichtbar sind die Blattfedern 18 seitlich im Bereich der Befestigungen der Spannbacken, die die Spannbackenzange öffnen, sobald der Druck auf den Pressbereich wieder gelöst wird.

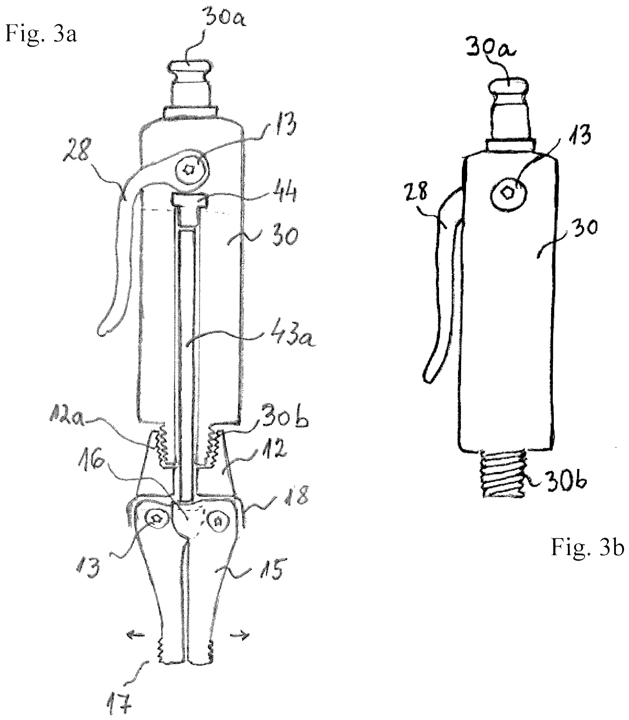

Figur 3a zeigt eine weitere beispielhafte Ausführungsform im Querschnitt. In diesem Fall ist der Werkzeugkopf ebenfalls mit zangenförmigen Spannbacken 15 ausgestattet, allerdings sind die Spannbackenschenkel hier annähernd gerade geformt und überkreuzen sich nicht. Ein Druck auf den Pressbereich 16, wie es oben für Figur 2 beschrieben wurde, sorgt daher für eine Bewegung der beiden Spannbacken 15 voneinander weg nach außen, wie durch Pfeile angedeutet ist. Aus diesem Grund sind auch die eingekerbten Haltebereiche 17 in diesem Fall auf der Außenseite der Spannbacken angeordnet.

Anstelle der Gleithammervorrichtung mit Führungsstange ist in dieser Ausführungsform ein Spannhandgriff 30 mit Pneumatikadapter 30a vorgesehen, so dass der Ausschlagimpuls oder eine pneumatische Zugkraft über ein angeschlossenes pneumatisches Werkzeug aufgebracht werden kann. Figur 3b zeigt diesen Spannhandgriff mit Pneumatikanschluss ohne angebrachten Werkzeugkopf in Draufsicht. Ansonsten ist der Handgriff 30 ähnlich ausgestaltet wie bei der vorhergehenden Ausführungsform der Figur 2. In dem Handgriff 30 ist eine innen liegende Ausnehmung eingebracht, die in Richtung des Werkzeugkopfs geöffnet ist. In der Ausnehmung verläuft eine Stellstange 43a, die durch eine Betätigung des Hebels 28 am oberen Ende in Richtung des Werkzeugkopfs bewegt werden kann und dort einen Druck auf den Pressbereich 16 der Spannbacken 15 auswirken kann, die sich infolge dessen nach außen bewegen. Wahlweise befindet sich wieder ein Druckstück 44 zum Kraftübertrag zwischen Stellstange 43a und Hebel 28. Dabei könnte der Handgriff 30 natürlich auch, ähnlich wie in Fig. 2, als Stange oder in anderer geeigneter Form ausgebildet sein.

Erfindungsgemäß kann der Werkzeugkopf, wie beispielsweise in Figuren 2 und 3 gezeigt, lösbar an dem Bedienteil angebracht sein, beispielsweise über ein Gewinde. In den gezeigten Figuren ist am Werkzeugkopf ein Innengewinde (11a, 12a) und am Bedienteil ein Außengewinde (12b, 20b, 29b, 30b) vorgesehen; natürlich können auch andere Gewindekombinationen vorliegen. Ebenso sind andere Befestigungsmöglichkeiten denkbar, beispielsweise Steckverbindungen, die optional zusätzlich durch Schrauben oder Klemmen fixiert werden. Ein spezielles Werkzeug für einen bestimmten Einsatzzweck könnte aber auch eine einstückige oder zumindest nicht lösbare Verbindung zwischen Werkzeugkopf und Bedienteil aufweisen.

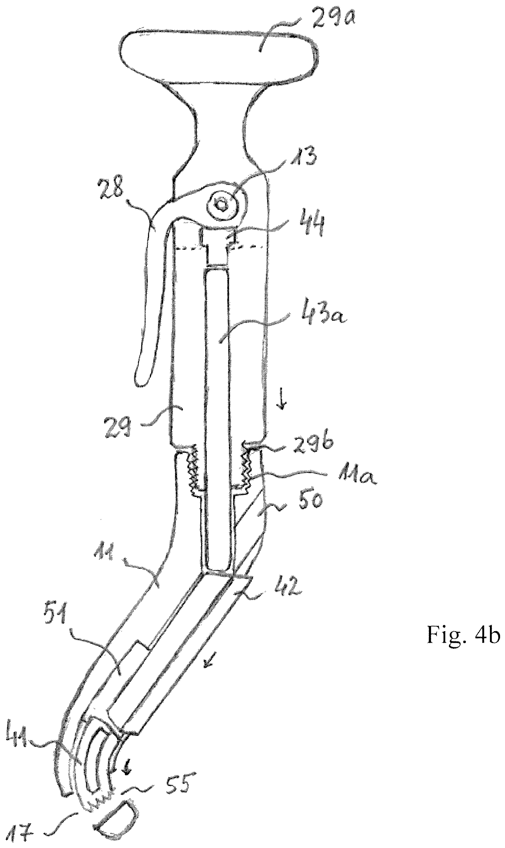

Figur 4a zeigt eine Draufsicht auf ein illustratives Beispiel, welches nicht unter den Schutzbereich des Anspruchs 1 fällt, Figur 4b zeigt dieselbe Werkzeugzusammensetzung im Querschnitt. Der Handgriff 29 hat keinen Pneumatikadapter wie in Figur 3, sondern weist statt dessen am vom Werkzeugkopf abgewandten Ende eine Schlagplatte 29a auf. Die übrigen Teile, Hebel 28 mit Befestigungsstift 13, Handgriff 29 mit Ausnehmung für die Stellstange 43a und die Funktionsweise des Bedienteils entsprechen der vorhergehenden Ausführungsform. Auf die Schlagplatte kann in geeigneter Weise ein Hammerschlag oder ein anders erzeugter Impuls aufgebracht werden, so dass der Impuls auf die eingespannte Prothese übertragen werden kann.

Der in Figur 4 gezeigte illustrative Werkzeugkopf dagegen ist nicht mit zangenartigen Spannbacken ausgestattet, sondern mit einer beweglichen Spannbacke 41, die gegen eine feststehende Spannbacke gepresst wird, welche als gekrümmte Verlängerung des Werkzeugkörpers 11 ausgebildet ist. Der Werkzeugkörper 11 ist ausgehend von dem Verbindungsbereich zwischen Bedienteil und Werkzeugkopf gekrümmt und weist eine Führung 50 für ein weiteres Stellglied 42 im Werkzeugkörper auf, das verschiebbar in dieser Führung gelagert ist. Im Anschluss daran ist die bewegliche Spannbacke in Form eines einsetzbaren Spannglieds in einer Öffnung 51 des Werkzeugkörpers verschiebbar eingebracht. Im Einsatz übt die Stellstange 43a bei Betätigung Druck auf das Stellglied 42 auf, welches wiederum den Druck auf die Spannbacke 41 weiter gibt, die so in Richtung des Endbereichs des Werkzeugkörpers gedrängt wird, wobei der Endbereich somit die zweite, feststehende Spannbacke bildet. Der Werkzeugkörper ist in diesem Bereich seitlich geöffnet 55a, um einen Prothesenteil, etwa den Prothesenhals einer Hüftprothese, aufzunehmen, wie in Figur 4a gezeigt ist. Das Ende der Spannbacke 41 kann auch hier mit Einkerbungen 17 für einen festeren Halt an dem Prothesenteil versehen sein. Sobald der Prothesenteil in der Öffnung 55a aufgenommen ist (die freiliegt, solange die Spannvorrichtung nicht betätigt wurde), kann durch Betätigung des Hebels am Bedienteil die Spannbacke 41 in Richtung der Öffnung gedrückt werden, um so den Prothesenteil zwischen dem Haltebereich der Spannbacke und dem Endbereich des Werkzeugkörpers einzuspannen.

Alle vorstehend gezeigten Bedienteile können mit den Werkzeugköpfen der bisherigen und folgenden Figuren beliebig kombiniert werden, also mit den Gleithammer-Bedienteilen ebenso wie mit dem Schlagplatten-Griff oder dem Pneumatik-Griff. Ebenso sind andere, hier nicht beschriebene Bedienteile für solche Werkzeugköpfe denkbar. Die Befestigung an Bedienteilen erfolgt wie auch in den vorstehenden Beispielen über beidseitige Gewinde oder andere stabile Befestigungsmechanismen. Ebenso könnten die Werkzeugköpfe der folgenden Figuren fest mit einem geeigneten Bedienteil verbunden sein. Natürlich können Werkzeugköpfe und Werkzeuge dieser Art nicht nur zum Ausschlagen von Endoprothesen, sondern beispielsweise auch abgebrochenen Schrauben angewendet werden.

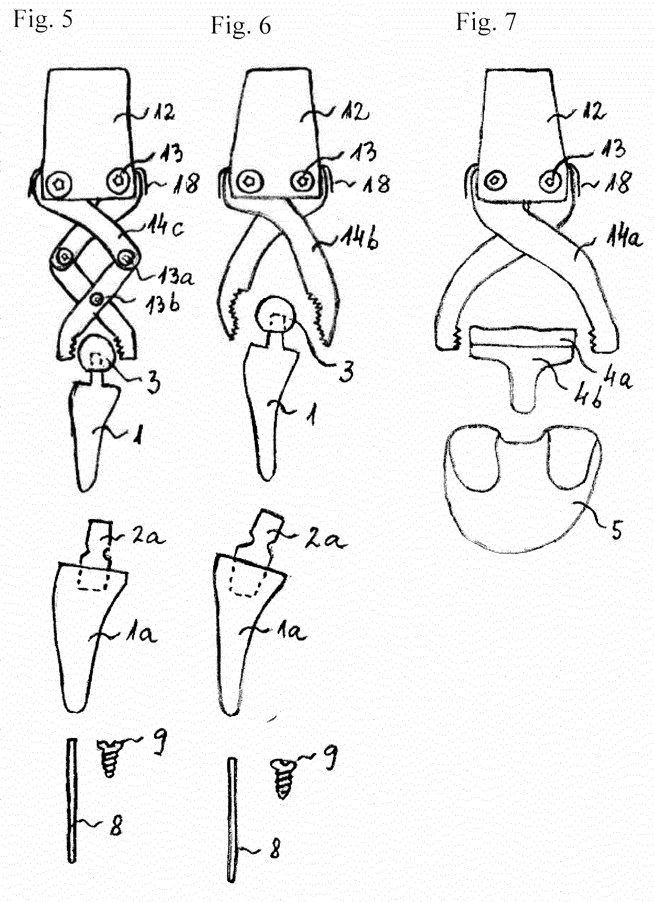

Figur 5 zeigt einen beispielhaften Werkzeugkopf der Erfindung ohne angebrachtes Bedienteil. Die Spannbacken 14c sind in diesem Beispiel zangenartig und bestehen aus mehreren beweglichen Abschnitten, die durch Stifte 13, 13a, 13b miteinander verbunden sind. Die Pressbereiche sind in diesem und den folgenden Beispielen nicht gezeigt, sind aber ebenso mittig zwischen den Befestigungsstiften 13 angelegt wie in den vorherigen Beispielen, so dass ein Druck auf den Pressbereich eine Bewegung der Spannbacken zur Folge hat. In Figur 5 umfassen die Spannbacken zwei scherenartig gekreuzte Zangenteile, an deren abgewinkeltem Ende die Haltebereiche mit Kerben zum Halten des Prothesenteils 3 vorgesehen sind. Diese beiden Teile sind mittig mit einem Stift 13b drehbar verbunden. Zwei weitere Zangen- bzw. Backenabschnitte sind zwischen diesen Endteilen und dem Werkzeugkörper mit Stiften 13a und 13 überkreuzt befestigt, so dass bei Druck auf einen nicht gezeigten Pressbereich zwischen den Befestigungsstiften 13 die beiden Spannbacken aufeinander zu bewegen und der Halt durch mechanischen Zug an dem Zangensystem noch verstärkt werden kann. Im gezeigten Beispiel sind als einzuspannende Prothesenteile der Kopf 3 einer modularen Femuralprothese 1 gezeigt, alternativ der Hals 2a einer modularen Prothese, eine Schraube 9 oder ein Marknagel 8.

Figur 6 zeigt ein weiteres zangenartiges Beispiel eines Werkzeugkopfes, bei dem am Werkzeugkörper 12 zwei gekreuzte, konkav gekrümmte Spannbacken 14b mittels Stiften 13 angebracht sind. Auch hier werden die Endbereiche der Spannbacken aufeinander zu bewegt, um so beispielsweise wieder den Kopf 3 einer Femuralprothese 1 oder alternativ einen Prothesenhals, eine Schraube 9 oder einen Nagel 8 zu fixieren.

Die zangenartige Formation der Spannbacken 14a in Figur 7 ähnelt der Ausführungsform, die in Figur 6 gezeigt ist; allerdings sind die Krümmung bzw. Abwinkelungen der Spannbacken 14a verändert und dadurch an die Abmessungen des zu greifenden Prothesenteils angepasst, das in diesem Beispiel die tibiale Komponente 4a, 4b einer Knieprothese darstellt. Es ist auch sichtbar, dass die Haltebereiche in Figur 6 gerundet ausgestaltet sind, um so optimal an den runden Prothesenkopf 3 angepasst zu sein, während hier in Beispiel 7 für die maximale Angriffskraft gerade Haltebereiche vorgesehen sind.

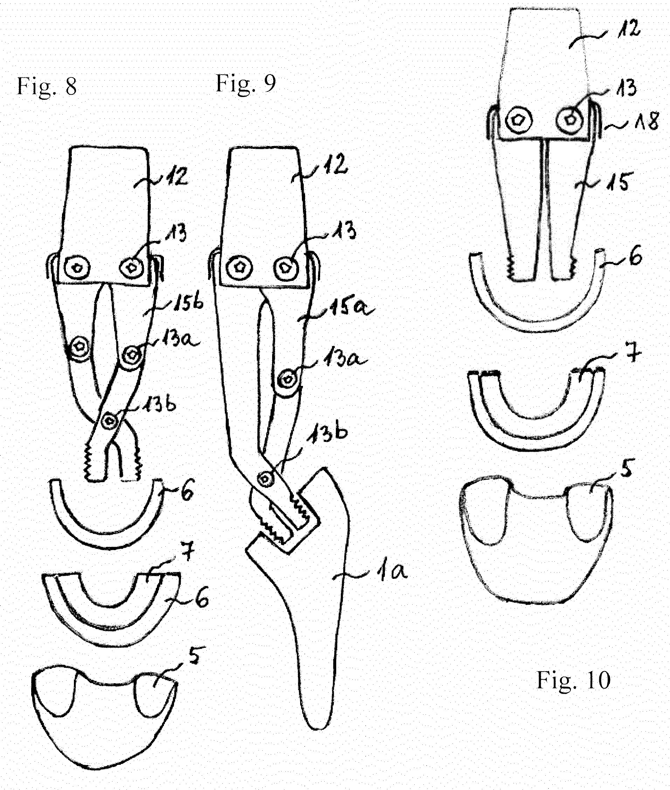

Figur 8 zeigt einen illustrativen beispielhaften Werkzeugkopf mit zangenartigen Spannbacken 15b, die jeweils aus zwei Abschnitten bestehen. Die unteren Spannbackenabschnitte sind gekreuzt und mittels eines Scharnierstifts 13b verbunden, während die daran anschließenden oberen Zangenabschnitte ungekreuzt mittels Stiften 13a bzw. 13 zwischen den unteren Abschnitten und dem Werkzeugkörper 12 angebracht sind. Dadurch ergibt sich bei Druckausübung auf den nicht gezeigten Druckbereich eine Bewegung der Spannbacken nach außen. Auf diese Weise kann in einen Hohlraum eingegriffen werden, beispielsweise wie gezeigt in ein Inlay 7 oder eine Pfanne 6 einer Hüftgelenksprothese, oder auch in die Wölbung der femuralen Komponente 5 einer Knieprothese. Die Einkerbungen im Haltebereich sind folgerichtig auf der Außenseite der Spannbacken angeordnet.

Figur 9 zeigt einen weiteren beispielhaften Werkzeugkopf, dessen Spannbacken 15a sich bei Druck nach außen bewegen und so in einen Hohlraum bzw. eine Ausnehmung eines Prothesenteils 1a eingreifen können. Hier sind die Bereiche der Spannbacke unterhalb des verbindenden Scharnierstifts 13b seitlich abgewinkelt, um optimal beispielsweise in die Ausnehmung des Schafts 1a einer modularen Hüftgelenksprothese eingreifen zu können. Eine Spannbacke ist einstückig ausgebildet und direkt über einen Stift 13 mit dem Werkzeugkörper 12 verbunden; die zweite Spannbacke umfasst zwei Abschnitte, die über einen Stift 13a miteinander verbunden sind. Dieser Aufbau ergibt sich aus der seitlichen Abwinkelung der Haltebereiche.

Figur 10 offenbart schließlich eine weitere illustrative Version eines Werkzeugkopfs, dessen Spannbacken sich nach außen bewegen. Die Spannbacken 15 sind im wesentlichen gerade ausgebildet und direkt und ungekreuzt mit dem Werkzeugkörper 12 verbunden. Auch eine solche Ausführungsform eignet sich beispielsweise zum Fixieren einer Pfanne 6 oder eines Inlays 7 einer Hüftgelenksprothese.

Es wird nochmals darauf hingewiesen, dass in jedem der beispielhaften Werkzeugköpfe der Figuren 5 bis 10 ein Bereich vorhanden ist, über den eine Bewegung der Stellstange 43, 43a in einem verbundenen Bedienteil auf die Spannbacken übertragen werden kann. Dies kann beispielsweise über einen Druckbereich bzw. Pressflügel mindestens einer Spannbacke geschehen, wie es in den Figuren 2 und 3 im Querschnitt gezeigt wurde.

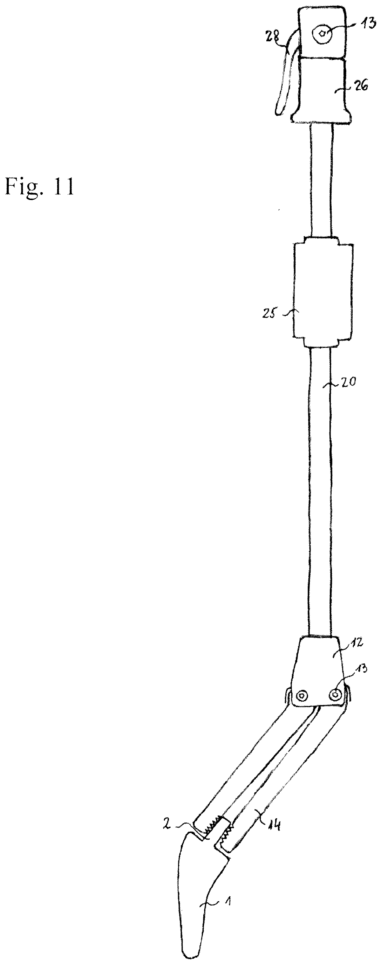

Figur 11 zeigt wieder ein Werkzeug mit Bedienteil und Werkzeugkopf. Der Bedienteil entspricht einem Gleithammer-Teil wie bei Figuren 1 und 2 ausführlich beschrieben. Der Werkzeugkopf umfasst zwei zangenartige bewegliche Spannbacken 14, die aber direkt nach der Stiftbefestigung 13 am Werkzeugkörper 12 gegenüber der senkrechten Werkzeugachse seitlich abgewinkelt sind. Auf diese Weise können sie bei Druck auf den nicht gezeigten Pressbereich sich aufeinander zu bewegen und den Prothesenhals 2 einspannen. Diese abgewinkelten Spannbacken sind vorzugsweise vorteilhaft für minimalinvasiven Anteriorlateralzugang geeignet.

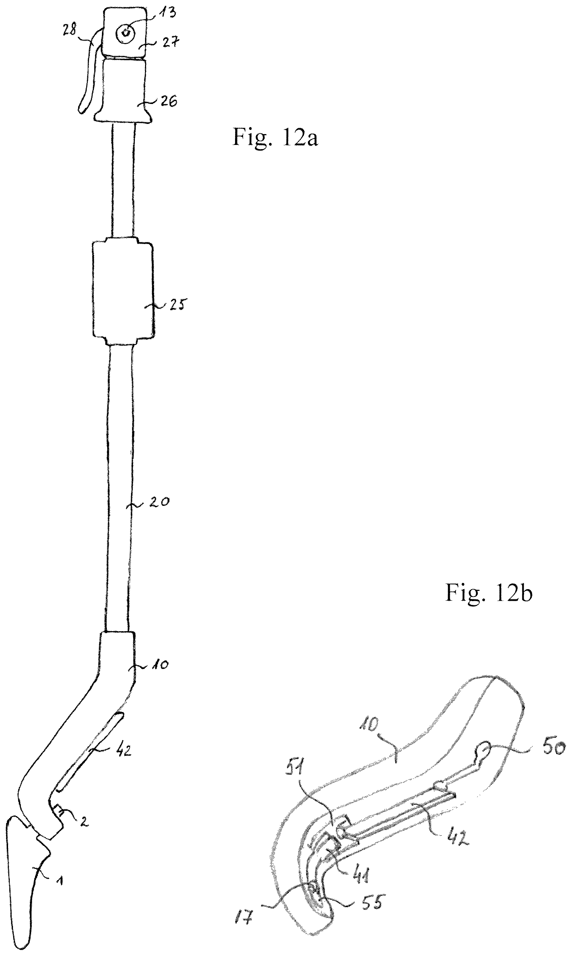

Figur 12a zeigt ein weiteres beispielhaftes Werkzeug für Hüftgelenksprothesen mit einem Gleithammerbedienteil wie in Figur 11. Der Werkzeugkörper 10 ist ähnlich ausgebildet wie der in Zusammenhang mit Figur 4 beschriebene Werkzeugkörper 11; allerdings ist der Werkzeugkörper 10 im Endbereich nicht zur Aufnahme eines Prothesenteils seitlich geöffnet, sondern geschlossen und weist im Endbereich ein Durchgangsloch 55 auf, das auf einen Prothesenhals aufgesetzt werden kann. Wie bei dem geöffneten Werkzeugkörper 11 wird dann eine in den Werkzeugkörper 10 eingesetzte Spannbacke 41 mit Einkerbungen 17 gegen den eingesetzten Prothesenhals gepresst und so zwischen dem Endbereich des Werkzeugkörpers, der als zweite Spannbacke dient, und der beweglichen Spannbacke 41 eingespannt. Eine genauere Schrägansicht dieser Ausführungsform eines geschlossenen Werkzeugkopfes 10 mit Durchgangsloch 55 ist in Figur 12b zu sehen. Zwischen der als verschiebbares Spannglied eingesetzten Spannbacke 41 und der Stellstange des Bedienteils ist wieder das verschiebbare Stellglied 42 eingesetzt, das verschiebbar in einer Gleitschiene oder Führung 50 des Werkzeugkörpers 10 gleitet und den Druck der Stellstange auf die Spannbacke 41 überträgt. Anstelle eines Prothesenhalses einer Femurprothese wie in Figur 12a gezeigt könnte ein ähnlicher Werkzeugkopf mit kleinerem Durchgangsdurchmesser beispielsweise andere Prothesenteile einspannen. Dieser Werkzeugkopf ist vorzugsweise vorteilhaft für minimalinvasiven Anteriorlateralzugang geeignet.

Dem Fachmann wird ersichtlich sein, dass die verschiedenen Zangen- und Spannvarianten hier zwar jeweils in Verbindung mit bestimmten Gelenkprothesen erwähnt und gezeigt wurden, dass damit aber die Anwendungsgebiete der Ausführungsformen nicht festgelegt sind. Der Operateur wird den Werkzeugkopf wählen, der für seinen konkreten Einsatz am besten geeignet ist; dies betrifft sowohl die Form der Prothese als auch die voraussichtlich notwendige Kraftaufwendung und den Winkel, in dem die Kraft aufgebracht werden muss. Der Fachmann wird außerdem in der Lage sein, ein Zangenwerkzeug so zu wählen oder zu entwerfen, dass über Schenkellängen und -winkel der Spannbacken die optimalen Kraftverhältnisse genutzt werden können, also einerseits die maximale Kraft zum Ausschlagen einer Prothese aufgebracht werden kann und die Anpresskraft der Spannbacken an der Prothese groß genug ist, um sie sicher zu fixieren, andererseits aber möglichst wenig Kraftübertrag auf den Knochen stattfindet und das Werkzeug möglichst leicht zu bedienen bleibt. Auch Zangensysteme nach dem Kniehebelprinzip sind beispielsweise als Werkzeugkopf denkbar, bei denen durch Zug eine besonders starke Fixierung der eingespannten Prothesenteile erfolgen kann. Der zangenartige Werkzeugkopf aus Figur 5 entspricht im wesentlichen diesem Prinzip.

Da das Werkzeug modular aus Bedienteil und Werkzeugkopf aufgebaut ist und die verschiedenen Teile nicht nur an den Gewinden, sondern auch einfach an den über Stifte oder Bolzen verbundenen Stellen auseinandergebaut werden können, wird eine einfache Reinigung des Werkzeugs ermöglicht, so dass alle Teile wieder verwendbar und auch austauschbar sind. Anstelle der Stifte könnten ebenso andere Scharniervarianten eingesetzt werden, etwa geschraubte Verbindungen, eingesetzte Scharnierelemente oder andere im Fach bekannte Lösungen. Ebenso ist es denkbar, insbesondere die zangenartigen Werkzeugköpfe, bei denen in den Beispielen die zwei Zangenteile durch einen Stift als Scharnier miteinander verbunden sind, stattdessen mit durchgestecktem Gewerbe auszuführen; also so, dass die Zangenschenkel bzw. Spannbackenschenkel nicht nur aufeinander gelegt sind, sondern ein Zangenteil durch eine Öffnung im anderen Zangenteil hindurchgeführt ist.

Ähnliches gilt für das Bedienelement 28, das in den Beispielen als Exzenterhebel dargestellt ist. Auch hier könnte ein anderes Element verwendet werden, das es ermöglicht, über Einwirkung auf die Stellstange die Spannbacken zuverlässig zu spannen. So ist es denkbar, die Stellstange mittels einer Feder zu spannen, optional auch in Verbindung mit einer Zahnstange und/oder einem Zahnrad. Vorzugsweise ist das Bedienelement verriegelbar und entriegelbar, so dass die Spannung aufrechterhalten bleibt, so lange dies gewünscht wird. Die Blattfedern 18, die zum Rückstellen der Spannbacken dienen, sind zwar hilfreich, aber für den erfinderischen Gedanken nicht zwingend notwendig. Sie könnten in speziellen Ausführungsformen auch weggelassen oder durch andere Rückstellelemente, etwa Spiralfedern oder ähnliches, ersetzt werden. Diese Alternativen könnten selbstverständlich in allen gezeigten Bedienteilen verwendet werden und mit allen Werkzeugköpfen des modularen Werkzeugs kombiniert werden.

## Patentansprüche

1. Werkzeugkopf für ein chirurgisches Werkzeug zum Ausschlagen und/oder Einsetzen von Prothesen, umfassend:
- einen Werkzeugkörper (10, 11, 12) mit mindestens zwei zangenartig ausgeführten Spannbacken (14, 14a, 14b, 14c, 15, 15a, 15b) zum Einspannen eines Prothesenteils, die relativ zum Werkzeugkörper (10, 11, 12) schwenkbar gelagert sind;
**dadurch gekennzeichnet, dass**
jede Spannbacke mit einem ersten Ende mittels eines Befestigungselements schwenkbar an dem Werkzeugkörper befestigt ist, und mindestens eine der beweglichen Spannbacken zumindest einen Druckbereich (16) benachbart zu dem ersten Ende zwischen den Befestigungselementen aufweist, der so angeordnet ist, dass eine Druckausübung auf den Druckbereich (16) der mindestens einen Spannbacke eine zur Richtung der Druckausübung im wesentlichen senkrechte Bewegung der Spannbacken (14, 14a, 14b, 14c, 15, 15a, 15b, 41) aufeinander zu bewirkt, um so das Prothesenteil zwischen Haltebereichen der Spannbacken einzuspannen.

2. Werkzeugkopf nach Anspruch 1, wobei die mindestens zwei Spannbacken (14a, 14b, 14c, 15a, 15b) überkreuzt angeordnet sind.

3. Werkzeugkopf nach einem der Ansprüche 1 bis 2, wobei mindestens eine der Spannbacken (14, 14a, 14b, 15a) relativ zur Werkzeugachse seitlich abgewinkelt ist.

4. Werkzeugkopf nach einem der Ansprüche 1 bis 3, wobei die Spannbacken (14, 14a, 14b, 14c) so angeordnet sind, dass sie sich bei Druckausübung auf den Druckbereich (16) nach innen aufeinander zu bewegen.

5. Werkzeugkopf nach einem der Ansprüche 1 bis 4, wobei mindestens eine der Spannbacken (14c, 15a, 15b) mehrere Abschnitte umfasst, die jeweils durch ein Stiftgelenk (13a) schwenkbar miteinander verbunden sind, wobei die Spannbackenabschnitte, welche die Haltebereiche umfassen, gekreuzt sind und mittels eines Stifts (13b) verbunden sind.

6. Werkzeugkopf nach einem der vorhergehenden Ansprüche, wobei an dem Werkzeugkörper (10, 11, 12) mindestens ein Rückstellelement (18) angeordnet ist, welches die Spannbacken (14, 14a, 14b, 14c, 15, 15a, 15b, 41) bei Beendigung der Druckausübung in ihre ursprüngliche Position zurückversetzt.

7. Werkzeugkopf nach einem der vorhergehenden Ansprüche, wobei mindestens eine der Spannbacken (14, 14a, 14b, 14c, 15, 15a, 15b, 41) in einem Haltebereich, der im Einsatz an einem einzuspannenden Prothesenteil anliegt, mit einem Profil (17) versehen ist.

8. Chirurgisches Werkzeug zum Ausschlagen und/oder Einsetzen von Prothesen, umfassend
einen Werkzeugkopf nach einem der vorhergehenden Ansprüche; und
einen Bedienteil, umfassend
ein Führungsgehäuse (20, 29, 30) mit einer axialen Ausnehmung, die mindestens in Richtung des Werkzeugkopfes geöffnet ist;
eine in dem Führungsgehäuse (20, 29, 30) axial verschiebbar gelagerte Stellstange (43, 43a),
ein Bedienelement (28),
und eine Schlagvorrichtung (30a, 29a, 25, 26), die dazu eingerichtet ist, einen auf das Bedienteil angewandten Impuls aufzunehmen oder aufzubringen und über das Werkzeug auf die Prothese zu übertragen;
wobei der Werkzeugkörper (10, 11, 12) des Werkzeugkopfes an dem Führungsgehäuse (20, 29, 30) des Bedienteils befestigt ist;
**dadurch gekennzeichnet, dass** das Bedienelement (28) so angeordnet ist, dass eine Betätigung des Bedienelements (28) eine Bewegung der Stellstange (43, 43a) in Richtung des Werkzeugkopfes bewirkt, wobei die Stellstange (43, 43a) so angeordnet ist, dass sie bei Betätigung einen Druck in axialer Richtung auf den Druckbereich (16) der mindestens einen beweglichen Spannbacke (14, 14a, 14b, 14c, 15, 15a, 15b, 41) ausübt.

9. Werkzeug nach Anspruch 8, wobei die Schlagvorrichtung eine Schlagplatte (29a) zum Aufnehmen eines mechanischen Schlags am werkzeugkopfabgewandten Ende des Bedienteils umfasst.

10. Werkzeug nach Anspruch 8, wobei die Schlagvorrichtung einen Gleithammer (25) umfasst, der beweglich auf dem Führungsgehäuse (20) gelagert ist, und einen Anschlag (26) für den Gleithammer (25) an einem oder beiden Enden des Führungsgehäuses.

11. Werkzeug nach Anspruch 8, wobei die Schlagvorrichtung einen Pneumatikadapter (30a) umfasst.

12. Werkzeug nach einem der Ansprüche 8 bis 11, wobei das Bedienelement einen Exzenterhebel (28) umfasst.

13. Werkzeug nach einem der Ansprüche 8 bis 12, wobei das Bedienelement (28) mit der Stellstange (43, 43a) indirekt über ein zwischenliegendes bewegliches Druckstück (44) in Verbindung steht.

14. Werkzeug nach einem der Ansprüche 8 bis 13, wobei der Werkzeugkopf lösbar durch ein Gewinde (11a, 12a, 29b) an dem Bedienteil befestigt ist.

## Claims

1. A tool head for a surgical tool for knockout and/or insertion of prostheses, comprising:
- a tool body (10, 11, 12) having at least two clamping jaws (14, 14a, 14b, 14c, 15, 15a, 15b) that are designed pliers-like for clamping a prosthetic part, the clamping jaws being pivotably mounted in relation to the tool body (10, 11, 12);
**characterized in that**
each clamping jaw is pivotably attached with a first end to the tool body by an attachment element, and at least one of the movable clamping jaws has at least one pressure area (16) adjacent to the first end between the attachment elements, which is arranged so that exerting a pressure on the pressure area (16) of the at least one clamping jaw causes a movement of the clamping jaws (14, 14a, 14b, 14c, 15, 15a, 15b, 41) toward one another to thereby clamp the prosthetic part between holding areas of the clamping jaws, wherein the movement of the clamping jaws is substantially perpendicular to the direction of exerting pressure.

2. The tool head according to Claim 1, wherein the at least two clamping jaws (14a, 14b, 14c, 15a, 15b) are arranged in a crossed fashion.

3. The tool head according to any one of Claims 1 through 2, wherein at least one of the clamping jaws (14, 14a, 14b, 15a) is bent sideward at an angle in relation to the tool axis.

4. The tool head according to any one of Claims 1 through 3, wherein the clamping jaws (14, 14a, 14b, 14c) are arranged so that they move inward toward one another when pressure is applied to the pressure area (16).

5. The tool head according to any one of Claims 1 through 4, wherein at least one of the clamping jaws (14c, 15a, 15b) comprises multiple sections, each of which is pivotably connected to one another by a pin joint (13a), wherein the sections of the clamping jaw, which comprise the holding areas, are crossed and are connected by a pin (13b).

6. The tool head according to any one of the preceding claims, wherein at least one restoring element (18) is arranged on the tool body (10, 11, 12), said restoring element returning the clamping jaws (14, 14a, 14b, 14c, 15, 15a, 15b, 41) to their original position at the end of the application of pressure.

7. The tool head according to any one of the preceding claims, wherein at least one of the clamping jaws (14, 14a, 14b, 14c, 15, 15a, 15b, 41) is provided with a profile (17) in a holding area which is in contact with a prosthetic part to be clamped during use.

8. A surgical tool for knockout and/or insertion of prostheses, comprising
a tool head according to any one of the preceding claims; and
an operating part, comprising
a guide housing (20, 29, 30) having an axial recess which is open at least in the direction of the tool head;
an adjusting rod (43, 43a), which is mounted to be axially displaceable in the guide housing (20, 29, 30),
an operating element (28),
and a striker mechanism (30a, 29a, 25, 26) which is equipped to receive or apply a momentum that is applied to the operating part and to transfer this momentum to the prosthesis by way of the tool;
wherein the tool body (10, 11, 12) of the tool head is attached to the guide housing (20, 29, 30) of the operating part;
**characterized in that** the operating element (28) is arranged so that operation of the operating element (28) causes a movement of the adjusting rod (43, 43a) in the direction of the tool head, wherein the adjusting rod (43, 43a) is arranged so that on actuation it exerts a pressure in axial direction on the pressure area (16) of the at least one movable clamping jaw (14, 14a, 14b, 14c, 15, 15a, 15b, 41).

9. The tool according to Claim 8, wherein the knocking device comprises a striker plate (29a) for receiving a mechanical impact on the end of the operating part away from the tool head.

10. The tool according to Claim 8, wherein the striker mechanism comprises a slide hammer (25) which is movably mounted on the guide housing (20) and a stop (26) for the slide hammer (25) is provided on one or both ends of the guide housing.

11. The tool according to Claim 8, wherein the striker mechanism comprises a pneumatic adapter (30a).

12. The tool according to any one of Claims 8 through 11, wherein the operating element comprises an eccentric lever (28).

13. The tool according to any one of Claims 8 to 12, wherein the operating element (28) is indirectly connected to the adjusting rod (43, 43a) via an intermediate movable pressure piece (44).

14. The tool according to any one of Claims 8 to 13, wherein the tool head is detachably attached to the operating element by a thread (11a, 12a, 29b).

## Revendications

1. Tête d'outil pour un outil chirurgical pour choquer et/ou insérer des prothèses, comprenant :
- un corps d'outil (10, 11, 12) comprenant au moins deux mâchoires de serrage (14, 14a, 14b, 14c, 15, 15a, 15b) conçues comme une pince pour serrer une partie de prothèse, qui sont disposées pivotantes par rapport au corps d'outil (10, 11, 12) ;
**caractérisée en ce que**
chaque mâchoire de serrage est fixée pivotante au corps d'outil par sa première extrémité au moyen d'un élément de fixation, et au moins une des mâchoires de serrage mobiles présente au moins une zone de pression (16) voisine de la première extrémité entre les éléments de fixation, qui est ainsi disposée qu'une pression exercée sur la zone de pression (16) de l'au moins une mâchoire de serrage provoque un déplacement des mâchoires de serrage (14, 14a, 14b, 14c, 15, 15a, 15b, 41) l'une sur l'autre essentiellement vertical au sens d'application de la pression, pour serrer ainsi la partie de prothèse entre des zones de retenue des mâchoires de serrage.

2. Tête d'outil selon la revendication 1, dans laquelle les au moins deux mâchoires de serrage (14a, 14b, 14c, 15a, 15b) sont disposées croisées.

3. Tête d'outil selon l'une des revendications 1 à 2, dans laquelle au moins une des mâchoires de serrage (14, 14a, 14b, 15a) est pliée latéralement par rapport à l'axe de l'outil.

4. Tête d'outil selon l'une des revendications 1 à 3, dans laquelle les mâchoires de serrage (14, 14a, 14b, 14c) sont ainsi disposées qu'elles se déplacent l'une sur l'autre vers l'intérieur lors de l'application de la pression sur la zone de pression (16).

5. Tête d'outil selon l'une des revendications 1 à 4, dans laquelle au moins une des mâchoires de serrage (14c, 15a, 15b) présente plusieurs tronçons qui sont reliés entre eux en étant pivotants respectivement par une articulation à tourillon (13a), dans lequel les tronçons de mâchoire de serrage qui comprennent les zones de retenue, sont croisés et reliés au moyen d'un tourillon (13b).

6. Tête d'outil selon l'une des revendications précédentes, dans laquelle au moins un élément de rappel (18) est disposé sur le corps d'outil (10, 11, 12), lequel renvoie les mâchoires de serrage (14, 14a, 14b, 14c, 15, 15a, 15b, 41) à leur position d'origine lorsque l'application de pression est terminée.

7. Tête d'outil selon l'une des revendications précédentes, dans laquelle au moins une des mâchoires de serrage (14, 14a, 14b, 14c, 15, 15a, 15b, 41) est dotée d'un profilé (17), dans une zone de retenue qui, en utilisation, repose contre une partie de prothèse à serrer.

8. Outil chirurgical pour choquer et/ou insérer des prothèses, comprenant
une tête d'outil selon l'une des revendications précédentes ; et
une partie de commande, comprenant
un logement de guidage (20, 29, 30) avec un évidement axial qui est ouvert au moins en direction de la tête d'outil ;
une tringle de commande (43, 43a) disposée dans le logement de guidage (20, 29, 30) en pouvant être déplacée axialement,
un élément de commande (28),
et un dispositif à choc (30a, 29a, 25, 26) qui est conçu pour recevoir ou fournir une impulsion appliquée sur la partie de commande et la transmettre à la prothèse par le biais de l'outil ;
dans lequel le corps d'outil (10, 11, 12) de la tête d'outil est fixé sur le logement de guidage (20, 29, 30) de la partie de commande ;
**caractérisé en ce que** l'élément de commande (28) est ainsi disposé qu'un actionnement de l'élément de commande (28) provoque un déplacement de la tringle de commande (43, 43a) en direction de la tête d'outil, dans lequel la tringle de commande (43, 43a) est ainsi disposée que lors de l'actionnement, elle exerce une pression dans le sens axial sur la zone de pression (16) de l'au moins une mâchoire de serrage mobile (14, 14a, 14b, 14c, 15, 15a, 15b, 41).

9. Outil selon la revendication 8, dans lequel le dispositif à choc comprend un plateau à choc (29a) pour recevoir un choc mécanique sur l'extrémité de la partie de commande détournée de la tête d'outil.

10. Dispositif selon la revendication 8, dans lequel le dispositif à choc comprend un marteau à inertie (25) qui est disposé mobile sur le logement de guidage (20) et une butée (26) pour le marteau à inertie (25) sur une extrémité ou les deux du logement de guidage.

11. Outil selon la revendication 8, dans lequel le dispositif à choc comprend un adaptateur pneumatique (30a).

12. Outil selon l'une des revendications 8 à 11, dans lequel l'élément de commande comprend un levier d'excentrique (28).

13. Outil selon l'une des revendications 8 à 12, dans lequel l'élément de commande (28) est en liaison indirecte avec la tringle de commande (43, 43a) par le biais d'une pièce de pression mobile (44) placée entre.

14. Outil selon l'une des revendications 8 à 13, dans lequel la tête d'outil est fixée à la pièce de commande de manière amovible par un filetage (11a, 12a, 29b).
